# EUROPÄISCHE PATENTSCHRIFT

(11) **EP 0 788 764 B1**
(45) Veröffentlichungstag und Bekanntmachung des Hinweises auf die Patenterteilung: **22.01.2003**
(21) Anmeldenummer: 97101577.1
(22) Anmeldetag: 01.02.1997
(51) Int. Cl.: A61B 1/00, A61B 1/227

(54) **Kupplung für ein Otoskop**
Coupling means for an otoscope
Accouplement pour un otoscope

(30) Priorität: 08.02.1996 DE 29602142 U
(43) Veröffentlichungstag der Anmeldung: 13.08.1997
(73) Patentinhaber: KIRCHNER & WILHELM GMBH & CO., D-71679 Asperg (DE)
(72) Erfinder: Kirchner, Regina, 71706 Markgröningen (DE)
(74) Vertreter: KOHLER SCHMID + PARTNER

(56) Entgegenhaltungen:
- DE-U- 8 709 151
- FR-A- 2 623 078
- GB-A- 2 130 091

## Beschreibung

Otoskope bestehen üblicherweise aus einem Halter und einem mit diesem verbindbaren Kopfteil, wobei das Kopfteil ausgewechselt werden kann. Der Halter ist als Handgriff und als Batterieträger ausgebildet, während das Kopfteil ein Sichtfenster und gegebenenfalls eine Beleuchtungseinheit aufweist. Die Erfindung bezieht sich auf eine Kupplung für ein solches Otoskop, um den Halter mit dem Kopfteil zu verbinden. Bei einem bekannten Gerät weist das Kopfteil einen mit einer oder mehreren Rastnuten versehenen Schaft auf, welcher in einen rohrförmigen Ansatz des Halters mit mindestens einem Rastglied einrastbar ist. Die Entrastung erfolgt durch eine am Aufsatz beweglich angeordnete Hülse. Dabei ist die Hülse axial unverschiebbar und in Umfangsrichtung drehbar auf dem Aufsatz angeordnet. Der Schaft weist außer der teilweise unterbrochenen Rastnut keilförmige Ansätze auf, welche mit dem mit der Hülse verbundenen gegen Federkraft verdrehbaren Rastglied so zusammenwirken. daß beim axialen Einschieben des Schaftes das Rastglied durch die Schrägflächen der keilförmigen Ansätze zurückgeschwenkt wird und so das Einsetzen des Schaftes ermöglicht. Sobald das Rastglied an die Nut zu liegen kommt, wird es durch die Federkraft in seine Sperrlage zurückgeschwenkt, so daß die Teile fest miteinander verbunden sind. Zum Lösen wird die Hülse von Hand in Umfangsrichtung gegen die Federkraft gedreht, so daß die Nut freigegeben wird und die Trennung der Teile erfolgen kann. Durch die Umlenkung der Axialbewegung in eine Rotation ergibt sich naturgemäß eine erhöhte Reibung, so daß zumindest beim Einsetzen relativ hohe Kräfte aufgewandt werden müssen. Auch ist die Herstellung des Schaftes als auch des Aufsatzes mit der Hülse sehr aufwendig.

Die Aufgabe der vorliegenden Erfindung ist es, einerseits eine sehr einfach herzustellende, jedoch ebenfalls sicher wirkende Kupplung zu schaffen, bei welcher die Verrastung ohne Umlenkung der Axialbewegung des Kopfteiles in eine Rotation der Hülse möglich ist. Dies wird erfindungsgemäß dadurch erreicht, daß als Rastglied ein Bolzen, Stift od. dgl. vorgesehen ist, welcher etwa quer zur Längsachse des Aufsatzes in einem von innen nach außen zum Halter hin verlaufenden Schrägschlitz des Aufsatzes gegen zum

Innenteil des Schlitzes gerichtete Federkraft gleitend gelagert ist, wobei die auf dem Aufsatz axial zum Halter hin verschiebbare Hülse auf dem Bolzen oder der Feder aufliegt.

Beim Einsetzen des Schaftes in den Aufsatz werden die Bolzen nach unten gedrückt, wobei sie sich gleichzeitig soweit schräg nach außen bewegen, bis der Bolzen mit der Innenwand des Aufsatzes abschließt. Sobald dann die Ringnut des Schaftes in die Höhe der Bolzen zu liegen kommt, können diese durch die Kraft der Feder in ihre ursprüngliche Lage zurückspringen und in die Ringnut einrasten, so daß die Teile fest miteinander verbunden sind. Zum Lösen ist lediglich die Hülse in Richtung zum Halter zu verschieben, wobei sie die Feder bzw. den Bolzen mitnimmt und dadurch der Bolzen die Ringnut des Schaftes freigibt, so daß dieser herausgezogen werden kann. Der Aufbau der erfindungsgemäßen Kupplung ist ausgesprochen einfach, da lediglich ein Schlitz, ein Bolzen und eine Feder erforderlich sind, um die Verrastung des Schaftes herbeizuführen. Auch die Reibungsverhältnisse sind relativ günstig, denn der waagrechte Weg, welchen die Bolzen zurücklegen müssen, ist minimal, so daß das Ver- und Entrasten der Teile sehr leicht zu handhaben ist.

Vorzugsweise dient zur Auflage der Hülse auf dem Bolzen mindestens eines seiner Enden, welches seitlich aus dem Schlitz herausragt. Um eine gleichmäßige Anlage der Teile zu erreichen, ist nach einem weiteren Merkmal der Erfindung zwischen dem Bolzen und dem Federende ein Zwischenring vorgesehen. Dieser Zwischenring kann sich dabei ebenfalls auf dem seitlich aus dem Schlitz herausragenden Bolzenende und zwar gegenüber der Auflagefläche der Hülse abstützen. Um eine Verkantung der Bolzen zu vermeiden, ragen zweckmäßig beide Enden der Bolzen seitlich aus dem Schlitz, wobei sich dann auch beiderseitige Auflagen des Zwischenrings und der Hülse ergeben.

Es können selbstverständlich beliebig viele Schrägschlitze und Bolzen Verwendung finden, jedoch ist es zweckmäßig lediglich zwei sich gegenüberliegende Schlitze mit Bolzen anzuordnen. Um die Hülse in ihrer Endlage zu sichern, ist der Aufsatz an seinem freien Ende mit einem in eine Ringnut eingreifenden Sprengring versehen. Um eine Art Auflauffläche zwischen dem Schaft und den Bolzen zu erreichen, ist der Schaft an seinem freien Ende mit einer schräg verlaufenden insbesondere abgerundeten Ringfläche versehen.

Die Zeichnung zeigt ein Ausführungsbeispiel der Erfindung. Es stellen dar:

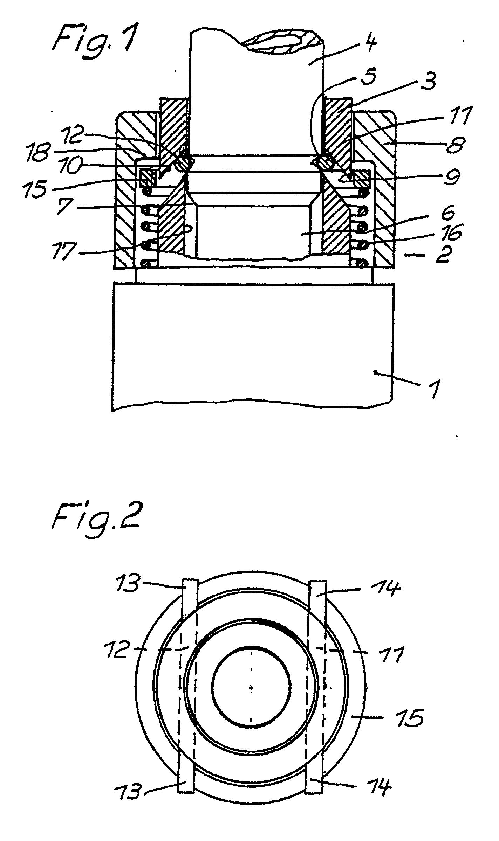

- Fig. 1: die teilweise abgebrochene und teilweise geschnittene Ansicht eines Halters mit dem abgebrochenen Schaft eines nicht dargestellten Kopfteiles eines Otoskop und
- Fig. 2: eine Draufsicht auf den Aufsatz nach Fig. 1 mit abgenommener Hülse.

Der Halter 1 des Otoskop ist an seinem oberen Ende durch eine Kappe 2 abgeschlossen. Diese Kappe 2 weist einen Aufsatz 3 auf, welcher zur Verbindung mit dem Schaft 4 eines nicht dargestellten Kopfteils des Otoskop dient. Der Schaft 4 ist mit einer Rastnut 5 versehen und an seinem freien Ende 6 besitzt er eine schrägverlaufende abgerundete Ringfläche 7, welche als Auflauffläche ausgebildet ist.

Der Aufsatz 3 ist rohrförmig ausgestaltet und auf ihm ist in axialer Richtung beweglich eine Hülse 8 angeordnet. Der Aufsatz 3 weist zwei von innen nach außen zum Halter 1 hin verlaufende Schrägschlitze 9 und 10 auf. In diesen Schrägschlitzen sind je ein Bolzen 11 und 12 quer zur Längsachse des Aufsatzes 3 gleitend gelagert, wobei die freien Enden 13 und 14 der Bolzen 11 und 12 aus den Schlitzen 9 und 10 seitlich herausragen. Unterhalb der Bolzen 11 und 12 ist an ihnen anliegend eine Ringscheibe 15 vorgesehen, welche sich mittels einer Schraubenfeder 16 auf der Kappe 2 abstützt, so daß die Bolzen 11 und 12 in der dargestellten oberen Lage in den Schlitzen 9 und 10 federnd gehalten werden.

Wenn der Schaft 4 in den rohrförmigen Aufsatz 3 eingesetzt wird, werden die Bolzen 11 und 12 durch das Schaftende 6 zunächst nach unten und gleichzeitig nach außen verschoben, bis sie mit der Innenseite 17 des Aufsatzes 3 bündig liegen. Durch die ringförmige Auflauffläche 7 des Schaftes 4 wird das Einsetzen desselben erleichtert. Sobald der Schaft 4 so weit eingeschoben ist, daß die

Ringnut 5 in die Höhe der Bolzen 11, 12 zu liegen kommt, rasten diese in die Ringnut ein, so daß der Schaft 4 fest in der Aufnahme 3 verrastet ist.

Die Hülse 8 weist an ihrem oberen Ende einen nach innen gerichteten ringförmigen Absatz 18 auf, mit welchem sie sich auf den seitlichen Enden 13 und 14 der Bolzen 11 und 12 abstützt. Wenn die Hülse 8 gegen die Kraft der Schraubenfeder 16 nach unten gedrückt wird, bewegen sich die Bolzen 11 und 12 ebenfalls nach unten und nach schräg außen, bis die Ringnut 5 des Schaftes 4 freigegeben ist und die Teile wiederum voneinander getrennt werden können.

## Patentansprüche

1. Kupplung für ein Otoskop zur Verbindung eines Halters mit einem Kopfteil, dessen mit einer oder mehreren Rastnuten versehener Schaft in einen rohrförmigen Aufsatz des Halters mit mindestens einem Rastglied einrastbar ist, wobei die Entrastung durch eine am Aufsatz beweglich angeordnete Hülse erfolgt, **dadurch gekennzeichnet, daß** als Rastglied ein Bolzen (11, 12), Stift od. dgl. vorgesehen ist, welcher etwa quer zur Längsachse des Aufsatzes (3) in einem von innen nach außen zum Halter (1) hin verlaufenden Schrägschlitz (9, 10) des Aufsatzes (3) gegen zum Innenteil des Schlitzes (9, 10) gerichtete Federkraft (16) gleitend gelagert ist, wobei die auf den Aufsatz (3) axial zum Halter (1) hin verschiebbare Hülse (8) auf dem Bolzen (11, 12) oder der Feder (16) aufliegt.

2. Kupplung nach Anspruch 1, **dadurch gekennzeichnet, daß** zur Auflage der Hülse (8) auf dem Bolzen (11, 12) mindestens eines seiner Enden (13, 14) seitlich aus dem Schlitz (9, 10) herausragt.

3. Kupplung nach einem oder beiden der vorhergehenden Ansprüche, **dadurch gekennzeichnet, daß** zwischen dem Bolzen (11, 12) und dem Federende (16) ein Zwischenring (15) vorgesehen ist.

4. Kupplung nach Anspruch 3, **dadurch gekennzeichnet, daß** sich der Zwischenring (15) an dem aus dem Schlitz (11, 12) heraustretenden Bolzenende (13, 14) abstützt.

5. Kupplung nach einem oder mehreren der vorhergehenden Ansprüche 2 bis 4, **dadurch gekennzeichnet, daß** der Bolzen (11, 12) mit beiden Enden (13, 14) seitlich aus dem Schlitz (9, 10) herausragt.

6. Kupplung nach einem oder mehreren der vorhergehenden Ansprüche, **dadurch gekennzeichnet, daß** der Aufsatz (3) mit zwei gegenüberliegenden Schrägschlitzen (9, 10) versehen ist.

7. Kupplung nach einem oder mehreren der vorhergehenden Ansprüche, **dadurch gekennzeichnet, daß** der Aufsatz (3) an seinem freien Ende zur Sicherung der Endlagerung der Hülse (8) in einer Ringnut einen Sprengring aufweist.

8. Kupplung nach einem oder mehreren der vorhergehenden Ansprüche, **dadurch gekennzeichnet, daß** der Schaft (4) an seinem freien Ende (6) mit einer schräg verlaufenden insbesondere abgerundeten Ringfläche (7) versehen ist.

## Claims

1. A coupling for an otoscope for the connection of a handle to a head part the shaft of which, which is provided with one or more locking grooves, can be locked into a tubular attachment of the handle by at least one locking member, unlocking being effected by a sleeve which is displaceably arranged on the attachment, **characterised in that** there is provided as the locking member a bolt (11, 12), pin or the like that is arranged in a sliding manner approximately at right angles to the longitudinal axis of the attachment (3) in an inclined slot (9, 10) of the attachment (3), which slot extends from the inside to the outside towards the handle (1), against a spring force (16) directed towards the inner part of the slot (9, 10), the sleeve (8), which is axially displaceable on the attachment (3) towards the handle (1), resting on the bolt (11, 12) or the spring (16).

2. A coupling according to claim 1, **characterised in that**, in order for the sleeve (8) to rest on the bolt (11, 12), at least one of the ends (13, 14) of the bolt projects laterally out of the slot (9, 10).

3. A coupling according to one or both of the preceding claims, **characterised in that** an intermediate ring (15) is provided between the bolt (11, 12) and the end of the spring (16).

4. A coupling according to claim 3, **characterised in that** the intermediate ring (15) is supported on the bolt end (13, 14) which extends out of the slot (9, 10).

5. A coupling according to one or more of the preceding claims 2 to 4, **characterised in that** the bolt (11, 12) projects laterally out of the slot (9, 10) by both ends (13, 14).

6. A coupling according to one or more of the preceding claims, **characterised in that** the attachment (3) is provided with two opposing inclined slots (9, 10).

7. A coupling according to one or more of the preceding claims, **characterised in that** the attachment (3) has at its free end a snap ring for securing the final position of the sleeve (8) in an annular groove.

8. A coupling according to one or more of the preceding claims, **characterised in that** the shaft (4) is provided at its free end (6) with an inclined, and especially rounded, annular surface (7).

## Revendications

1. Raccord pour un otoscope pour relier un manche avec une partie de tête, dont la tige dotée d'une ou plusieurs rainures d'arrêt peut s'enclencher dans une embase en forme de tuyau du manche avec au moins un élément d'arrêt, le désenclenchement se faisant par une gaine disposée de façon mobile sur le support, **caractérisé en ce que** comme élément d'arrêt est prévue une cheville (11, 12), broche ou similaire, qui est située de façon coulissante à l'encontre de la force d'un ressort (16) orienté vers la paroi interne de la fente (9, 10), à peu près en travers de l'axe longitudinal du support (3) dans une fente inclinée (9, 10) de l'intérieur vers l'extérieur en direction du manche (1), moyennant quoi la gaine (8) coulissant axialement sur le support (3) en direction du manche (1) repose sur la cheville (11, 12) ou le ressort (16).

2. Raccord selon la revendication 1, **caractérisé en ce que** pour l'appui de la gaine (8) sur la cheville (11, 12) au moins une de ses extrémités (13, 14) fait saillie sur le côté de la fente (9, 10).

3. Raccord selon l'une quelconque ou les deux revendications précédentes, **caractérisé en ce qu'**entre la cheville (11, 12) et l'extrémité du ressort (16) est prévu un anneau intermédiaire (15).

4. Raccord selon la revendication 3, **caractérisé en ce que** l'anneau intermédiaire (15) s'appuie sur l'extrémité de la cheville (13, 14) qui sort de la fente (11, 12).

5. Raccord selon l'une ou plusieurs quelconques revendications précédentes 2 à 4, **caractérisé en ce que** la cheville (11, 12) fait saillie de la fente (9, 10) par ses deux extrémités (13, 14).

6. Raccord selon l'une ou plusieurs quelconques revendications précédentes, **caractérisé en ce que** le support (3) est doté de deux fentes inclinées opposées (9, 10) .

7. Raccord selon l'une ou plusieurs quelconques revendications précédentes, **caractérisé en ce que** le support (3) présente à son extrémité libre un jonc dans une encoche circulaire pour sécuriser la position finale de la gaine (8).

8. Raccord selon l'une ou plusieurs quelconques revendications précédentes, **caractérisé en ce que** la tige (4) présente à son extrémité libre (6) une surface circulaire (7) inclinée et notammen arrondie.
